# EUROPEAN PATENT SPECIFICATION

(11) **EP 0 618 917 B1**
(45) Date of publication and mention of the grant of the patent: **26.03.1997**
(21) Application number: 93900708.4
(22) Date of filing: 25.11.1992
(51) Int. Cl.: C07D 471/10, A61K 31/435

(54) **1'-AMINO-2- (BENZOTHIAZOLYL)METHYL]SPIRO ISOQUINOLINE-4(1H),3'-PYRROLIDINE]-1,2',3,5'(2H)-TETRONES AND ANALOGS THEREOF USEFUL AS ALDOSE REDUCTASE INHIBITORS**
1'-AMINO-2[(BENZOTHIAZOLYL)METHYL] SPIRO [ISOCHINOLIN-4(1H), 3'-PYRROLIDINE]-1,2',3,5'-TETRONE UND ANALOGE DAVON VERWENDBAR ALS ALDOSEREDUKTASE LUHIBITOREN
1'-AMINO-2 (BENZOTHIAZOLYLE)METHYL]SPIRO ISOQUINOLINE-4(1H),3'-PYRROLIDINE]-1,2',3,5'(2H)-TETRONES ET LEURS ANALOGUES UTILES COMME INHIBITEURS DE REDUCTASE D'ALDOSE

(30) Priority: 25.11.1991 US 797567; 25.11.1991 US 798294
(43) Date of publication of application: 12.10.1994
(73) Proprietor: AMERICAN HOME PRODUCTS CORPORATION, Madison, New Jersey 07940-0874 (US)
(72) Inventor: MALAMAS, Michael, Sotirios, Jamison, PA 18929 (US)
(74) Representative: Wileman, David Francis, Dr.
(86) International application number: US9210268
(87) International publication number: WO9311126

(56) References cited:
- WO-A-92/06974
- WO-A-92/06975
- US-A- 5 037 831

## Description

This invention relates to 1'-amino-2-[(benzothiazolyl)methyl)spiro[isoquinoline-4(1H),3'-pyrrolidine]-1,2',3,5'(2H)-tetrones and analogs thereof and their pharmaceutically acceptable salts , to processes for their preparation, to methods for using the compounds, and to pharmaceutical preparations thereof. The compounds have pharmaceutical properties which render them beneficial for the prevention or treatment of complications associated with diabetes mellitus.

The use of insulin and/or oral hypoglycemic agents in the treatment of diabetes mellitus has prolonged the life of many of these patients. However, their use has not had a demonstrable impact on the development of diabetic complications such as neuropathy, nephropathy, retinopathy, cataracts and vascular disease which accompany the underlying metabolic disorder. There is little question that chronic hyperglycemia plays a major role in the genesis of these complications, and that complete normalization of blood glucose would likely prevent most if not all complications. For a number of reasons, though, chronic normalization of blood glucose has not been achieved with the currently available therapies.

The long-term complications of diabetes develop in tissues where glucose uptake is independent of insulin. In these tissues, which include the lens, retina, kidney and peripheral nerves, the systemic hyperglycemia of diabetes is rapidly transposed into high tissular concentrations of glucose. In all of these tissues this excess glucose is rapidly metabolized by the sorbitol pathway. The intense diabetes-induced flux of glucose through this pathway appears to initiate a cascade of biochemical alterations which slowly progress to cell dysfunction and structural damage. Aldose reductase, the key enzyme in the sorbitol pathway, reduces glucose to sorbitol at the expense of the cofactor NADPH. In animal models of diabetes, compounds which inhibit aldose reductase have been shown to prevent the biochemical, functional and morphological changes induced by hyperglycemia. Early studies by J. H. Kinoshita and collaborators implicated aldose reductase in the etiology of diabetic cataracts. More recent studies have provided compelling evidence that aldose reductase also plays a significant role in the initiation of diabetic nephropathy, retinopathy and neuropathy (cf McCaleb et al, J Diab. Comp., 2, 16, 1989; Robinson et al, Invest. Ophthalmol. Vis. Sci., 30., 2285, 1989; Novvest and Inserra, Diabetes, 36, 500, 1987).

### Prior Art

The closest prior art is Malamas US Patent 5,037,831, August 6, 1991; Malamas US Patent 5,045,544, September 3, 1991 and Malamas USSN 07/596,266 and 07/596,889 both filed October 11 1990 now published as WO 92/06975 and WO 92/06974 respectively. US Patent No 5037831 discloses spiroisoquinolinepyrrolidines unsubstituted on the pyrroline nitrogen which possess aldose reductase inhibiting properties. USSN 07/596,266 and 07/596,889 disclose (inter alia) the spiro-isoquinoline-pyrrolidine tetrones of formula 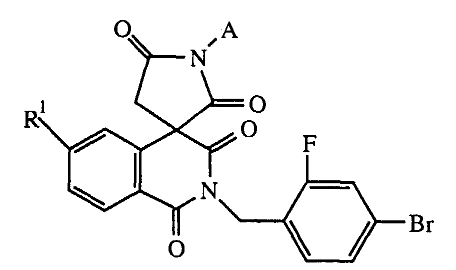 wherein:
A is -NR⁴R⁵ or -N=CR^{4'}R^{5'},
R¹ is hydrogen or fluorine, R⁴, R⁵ are hydrogen, acyl, carboalkoxy or trifluoromethanesulfonyl and R^{4'} and R^{5'} are alkyl or joined to form alicyclic or heterocyclic rings, useful as aldose reductase inhibitors for treating diabetic complications and galactosemia.

The 1'-amino-2-[(benzothiazolyl)methyl]spiro[isoquinoline-4(1H),3'-pyrrolidine]-1,2',3,5'(2H)-tetrones and analogs thereof of the present invention are represented by formula (I): 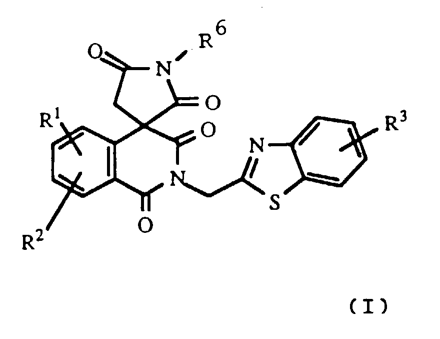 wherein:
R¹,R² and R³ are independently hydrogen, alkyl containing 1 to 6 carbon atoms, halogen, lower alkoxy containing 1 to 6 carbon atoms, trifluoromethyl or nitro;
R⁶ is 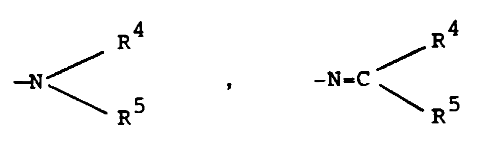 or 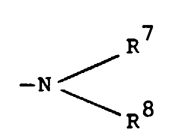 wherein
R⁴ and R⁵ are independently hydrogen, alkyl containing 1 to 6 carbon atoms, aryl, aryl (lower alkyl) wherein aryl contains 6 to 10 carbon atoms and lower alkyl contains 1 to 6 carbon atoms, alkanoyl of 2 to 5 carbon atoms or carboalkoxy, wherein alkoxy contains 1 - 6 carbon atoms or R⁴ and R⁵ are joined to form alicyclic or heterocyclic rings selected from the group consisting of 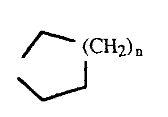 wherein n = 1-10; 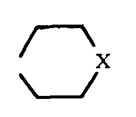 wherein X = O, S, SO, SO₂; 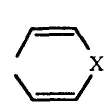 wherein X = O, S; and 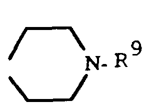 wherein R⁹ = H, lower alkyl containing 1 to 6 carbon atoms, aryl or aryl (lower alkyl)wherein aryl contain 6 to 10 carbon atoms and lower alkyl contains 1 to 6 carbon atoms,
and
R⁷ and R⁸ are independently alkylsulfoxy, arylsulfoxy, alkysulfonyl, arylsulfonyl wherein aryl contains 6 to 10 carbon atoms and alkyl contains 1 to 6 carbon atoms or R⁸ may also represent one of the values for R⁵ defined above, and the pharmaceutically acceptable salts thereof.
Examples of alkyl used herein as a group or part of a group such as alkoxy are straight or branched chains including methyl, ethyl, propyl, isopropyl and butyl. Examples of aryl are phenyl and naphthyl. Examples of aryl lower alkyl are benzyl, phenethyl, 3-phenylpropyl, 2-phenylpropyl and naphthylmethyl. Examples of R¹ and/or R² are hydrogen and halogen e.g fluorine such as 6-Fluoro. Examples of R³ are CF₃ e.g 5-CF₃. When R⁶ is -NR⁴R⁵ examples of R⁴ and/or R⁵ are hydrogen and alkanoyl, e.g acetyl. When R⁶ is -N-CR⁴R⁵ examples of R⁴ and/or R⁵ are alkyl of 1 - 3 carbon atoms such as methyl or ethyl, or R⁴ and R⁵ are joined to form rings such as

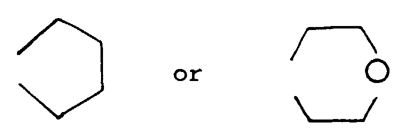

A preferred group of compounds is represented by formula (II): 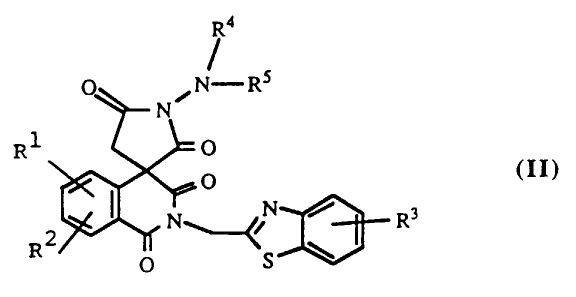 wherein:
R¹ and R² are hydrogen or halogen; R³ is trifluoromethyl, R⁴ and R⁵ are hydrogen or acetyl.

The most preferred compounds of formula II are set forth below:
1'-amino-2-[[(5-trifluoromethyl)-2-benzothiazolyl]methyl]spiro[isoquinoline-4(1H), 3'-pyrrolidine]-1,2',3,5'(2H)-tetrone and the pharmaceutically acceptable salts thereof;
1'-amino-6-fluoro-2-[[5-(trifluoromethyl)-2-benzothiazolyl]methyl]spiro[isoquinoline-4(1H), 3'-pyrrolidine]-1,2',3,5'(2H)-tetrone and the pharmaceutically acceptable salts thereof; and
N-[2-[[5-(trifluoromethyl)-2-benzothiazolyl]methyl]-2,3-dihydro-1,2',3,5'-tetraoxospiro[isoquinoline-4(1H), 3'-pyrrolidine]-1'-yl]acetamide.
Another preferred group of compounds of this invention are represented by formula (IIa): 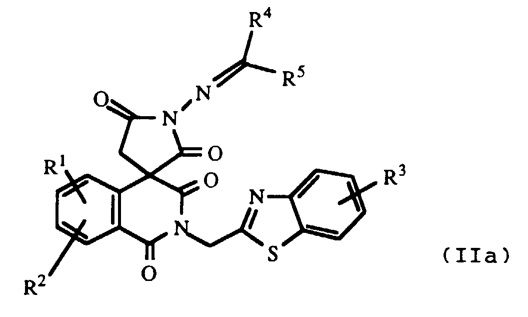 wherein:
R¹ and R² are hydrogen or halogen; R³ is trifluoromethyl; R⁴ and R⁵ are lower alkyl containing 1 to 3 carbon atoms or R⁴ and R⁵ are joined to form alicyclic or heterocyclic rings selected from the group consisting of 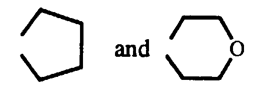 The most preferred compounds of formula IIa are set forth below:
1'-[(1 -methylethylidene)amino]-2-[[5-(trifluoromethyl)-2-benzothiazolyl]methyl]spiro[isoquinoline-4(1H),3'-pyrrolidine]-1,2',3,5'(2H)-tetrone;
1'-(cyclopentylideneamino)-2-[[5-(trifluoromethyl)-2-benzothiazolyl]methyl]spiro[isoquinoline-4(1H),3'-pyrrolidine]-1,2',3,5'(2H)-tetrone; and
1'-[(tetrahydro-4H-pyran-4-ylidene)amino]-2-[[5-(trifluoromethyl)-2-benzothiazolyl]methyl]spiro[isoquinoline-4(1H),3'-pyrrolidine]-1,2',3,5'(2H)-tetrone.

The compounds of formula (I) all possess at least one asymmetric carbon atom, namely the spiro carbon atom at position 3' of the pyrrolidine ring. The compounds of formula (I) therefore exist, and may be isolated, in two or more stereoisomeric forms. This invention encompasses the compounds of formula (I) in racemic form or in any optically active form.

A method is provided for preventing or relieving diabetes mellitus associated complications in a diabetic mammal by administering to said mammal a prophylactic or alleviating amount of the compounds of formula (I). Such complications include neuropathy, nephropathy, retinopathy, keratopathy, diabetic uveitis, cataracts and limited joint mobility.

The compounds of formula (I) and their pharmaceutically acceptable salts when admixed with a pharmaceutically acceptable carrier, form a pharmaceutical composition which can be used according to the preceding method.

The 1'-amino -2-[(benzothiazolyl)methyl]spiro[isoquino1ine-4(1H),3'-pyrrolidine]-1,2'3,5'(2H)-tetrones and analogs of this invent ion may be administered to mammals, for example, man, cattle, or rabbits either alone or in dosage forms, i.e capsules or tablets, combined with pharmacologically acceptable excipients.

The compounds of this invention may be given orally. However, the method of administering the present active ingredients of this invention is not to be construed as limited to a particular mode of administration. For example, the compounds may be administered topically directly to the eye in the form of drops of sterile, buffered ophthalmic solutions, preferably of pH 7.2-7.6. Also, they may be administered orally in solid form containing such excipients as starch, milk sugar, certain types of clay and so forth. They may also be administered orally in the form of solutions or they may be injected parenterally. For parenteral administration, they may be used in the form of a sterile solution, preferably of pH 7.2-7.6, containing a pharmaceutically acceptable buffer.

The dosage of the 1'-amino-2-[(benzothiazolyl)methyl]spiro[isoquinoline-4(1H),3'-pyrrolidine]-1,2',3,5'(2H)-tetrones will vary with the form of administration and the particular compound chosen. Furthermore, it will vary with the particular host under treatment. Generally, treatment is initiated with small dosages substantially less than the optimal dose of the compound. Thereafter, the dosage is increased by small increments until efficacy is obtained. In general, the compounds of this invention are most desirably administered at a concentration level that will generally afford effective results without causing any harmful or deleterious side effects. For topical administration, a 0.05-1.0% solution may be administered dropwise in the eye. The frequency of instillation varies with the subject under treatment from a drop every two or three days to once daily. For oral or parenteral administration a preferred level of dosage ranges from about 50 mg to about 100 mg per kilo of body weight per day, although aforementioned variations will occur. However, a dosage level that is in the range of from about 50 mg to about 100 mg per kilo of body weight per day is most satisfactory.

Unit dosage forms such as capsules, tablets, pills and the like may contain from about 50 mg to about 250 mg of the active ingredients of this invention with a pharmaceutical carrier. Thus, for oral administration, capsules can contain from between about 50 mg to about 250 mg of the active ingredients of this invention with or without a pharmaceutical diluent. Tablets, either effervescent or noneffervescent, can contain between about 50 to 250 mg of the active ingredients of this invention together with conventional pharmaceutical carriers. Thus tablets, which may be coated and either effervescent or noneffervescent, may be prepared according to the known art. Inert diluents or carriers, for example, magnesium carbonate or lactose, can be used together with conventional disintegrating agents for example, magnesium stearate.

The 1'-amino-2-[(benzothiazolyl)methyl]spiro[isoquinoline-4(1H),3'-pyrrolidine]-1,2',3,5'(2H)-tetrones and analogs can also be used in combination with insulin or oral hypoglycemic agents to produce a beneficial effect in the treatment of diabetes mellitus. In this instance, commercially available insulin preparations or oral hypoglycemic agents, exemplified by acetohexamide, chlorpropamide, tolazamide, tolbutamide and phenformin, are suitable. The compounds hereof can be administered sequentially or simultaneously with insulin or the oral hypoglycemic agent. Suitable methods of administration, compositions and doses of the insulin preparation or oral hypoglycemic agent are described in medical textbooks; for instance, *Physicians' Desk Reference,* 42 ed., Medical Economics Co., Oradell, N.J., U.S.A., 1988.

The aldose reductase inhibiting property of the compounds of this invention and the utilization of the compounds in preventing, diminishing and alleviating diabetic complications are demonstrable in experiments using galactosemic rats, see Dvornik *et al.,* Science, *182,* 1146 (1973). Such experiments are exemplified hereinbelow after the listing of the following general comments pertaining to these experiments:
(a) Four or more groups of six male rats, 50-70 g, Sprague-Dawley strain, were used. The first group, the control group, was fed a mixture of laboratory chow (rodent Laboratory Chow, Purina) and glucose at 20% (w/w %) concentration. An untreated galactosemic group was fed a similar diet in which galactose was substituted for glucose. The third group was fed a diet prepared by mixing a given amount of the test compound with the galactose containing diet The concentration of galactose in the diet of the treated groups was the same as that for the untreated galactosemic group.
(b) After four days, the animals were killed by euthanization. Both the lens and sciatic nerve were removed, weighed and stored frozen for polyol determination.
(c) The polyol determination was performed by a modification of the procedure of M. Kraml and L. Cosyns, Clin. Biochem., 2, 373 (1969). Only two minor reagent changes were made: (a) the rinsing mixture was an aqueous 5% (w/v) trichloroacetic acid solution and (b) the stock solution was prepared by dissolving 25 mg of dulcitol in 100 mL of an aqueous trichloroacetic acid solution. [N.B.: For each experiment the average value found in the tissue from rats fed the glucose diet was subtracted from the individual values found in the corresponding tissue in galactose-fed rats to obtain the amount of polyol accumulated.] The aldose reductase inhibiting effects of the compounds of formula (I) were also tested by employing an *in vitro* testing procedure similar to that described by S. Hayman and J.H. Kinoshita, J. Biol. Chem., 240, 877 (1965). In the present case the procedure of Hayman and Kinoshita was modified in that the final chromatography step was omitted in the preparation of the enzyme from bovine lens.

The following tabulated results (TABLES 1 and 2) show that the 1'-amino-2-[(benzothiazolyl)methyl]spiro[isoquinoline-4(1H),3'-pyrrolidine]-1,2',3,5'(2H)-tetrones and analogs of this invention show the property that they are active in vivo and diminish the accumulation of dulcitol in the lenses, sciatic nerves and diaphragm of rats fed galactose. The figures under L, N, and D represent the percentage decrease of dulcitol accumulation in the tissues of the lens, sciatic nerve, and diaphragm, respectively for treated rats as compared to untreated rats.

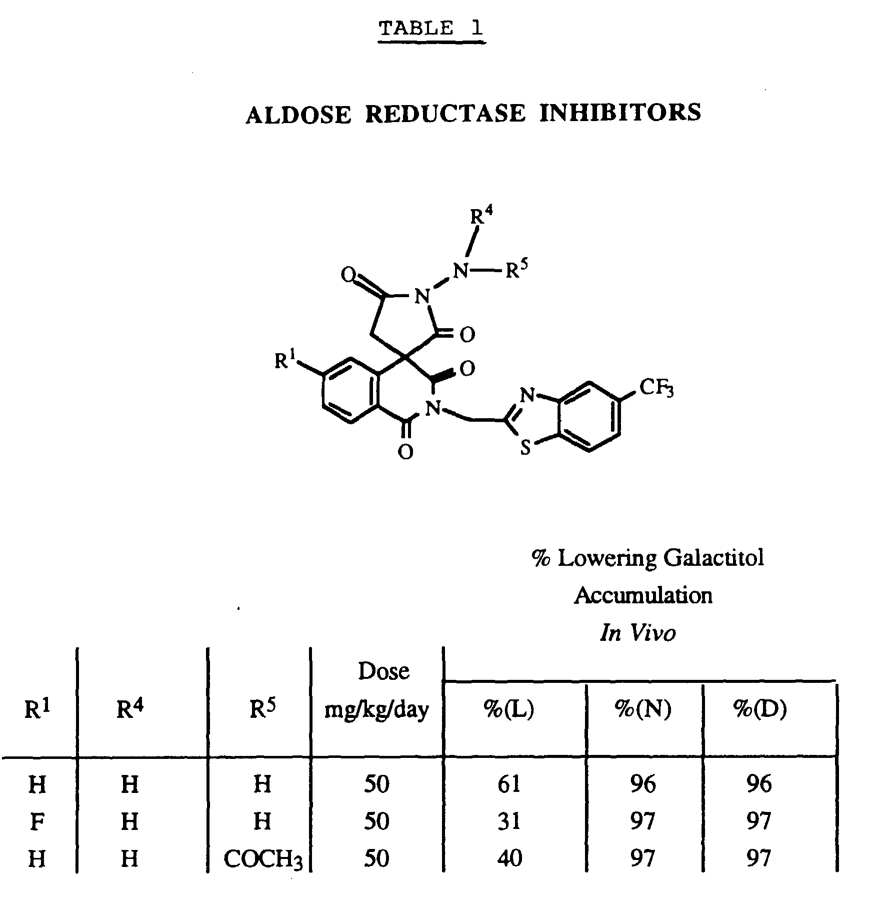

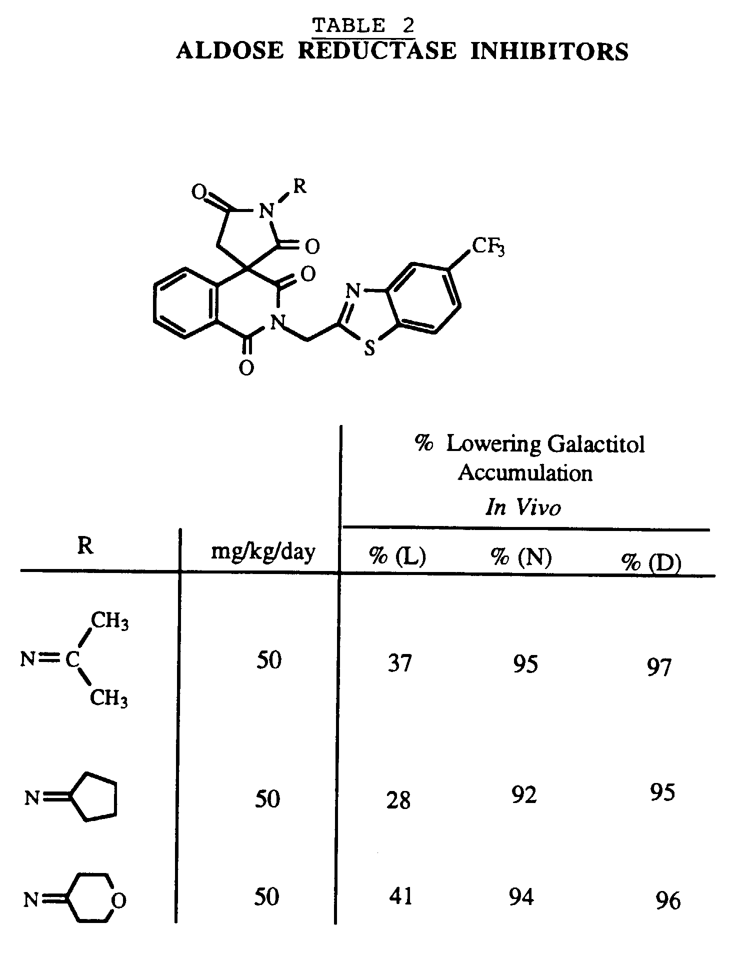

This invention also provides processes for preparing the compounds of formula I or salts thereof. In particular the compounds of formula I may be prepared by one of the following:
a) acylating a compound of formula (A) or (B)

   R⁴R⁵NNH₂ (A)

   R⁸R⁷NNH₂ (B)

   wherein R⁴, R⁵, R⁸ and R⁷ are as defined above with a compound of formula (C) 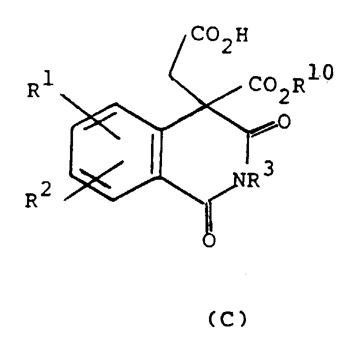 or an activated form thereof
   wherein CO₂R¹⁰ and is an ester function and e.g an alkyl or aralkyl ester such as C₁-C₆ alkyl ester, .eg methyl and R¹, R² and R³ are as defined above to give a corresponding compound of formula I wherein R⁶ is -NR⁴R⁵ or -NR⁸R⁷ ,
   or
b) acylating a compound of formula I wherein R⁶ is -NHR⁸ wherein R⁸ is as defined above with an acylating agent (including sulfonylating and sulfinylating agents) containing the group

   R¹¹CO-, R¹²OCO- or R¹³S(O)ₘ-

   where R¹¹ is C₁-C₄ alkyl, R¹² is C₁-C₆ alkyl and R¹³ is C₁-C₆ alkyl or C₆-C₁₀ aryl to give a corresponding compound of formula I wherein R⁶ is NR⁴R⁵ or -NR⁷R⁸
   wherein R⁵ and R⁸ are as defined hereinabove, R⁴ is alkanoyl or carboalkoxy, R⁷ is alkylsulfoxy, arylsulfoxy, alkylsulfonyl or arylsulfonyl and m is 1 or 2,
   or
c) reacting a compound of formula I wherein R⁶ is with a carbonyl compound of formula (D): 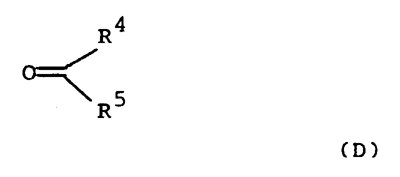 wherein R⁴ and R⁵ are as defined above, or a reactive derivative thereof, e.g an acetal, to give a compound of formula I wherein R⁶ is

   -N=CR⁴R⁵

   and if desired after any of the aforementioned processes isolating the product as a salt.

With regard to process a) the acylation may be carried out using the carboxylic acid of formula (C) and a coupling agent such as a carbodiimide e.g dicyclohexylcarbodiimide. Alternatively the carboxylic acid group may be in activated form e.g as an acid halide such as the chloride or bromide, or an anhydride such as a mixed anhydride. Processes for preparing compounds of formula (C) are described in publication No GB 2224734 and EP Publication No 365324.

With regard to process b) examples of the acylating agent are acid halides and anhydrides e.g compounds of formula R¹¹ COhal, (R¹¹CO)₂O, R¹³S(O)ₘhal, (R¹³SO₂)₂O and haloformates such as Cl COOR¹².

Sequential acylation may be preformed using different acylating agents. Multiple acylation may be effected using a stoichiometric excess of acylating agent and more vigorous acylating conditions.

With regard to process c) the reaction may be carried out in conventional manner for preparing Schiff bases. Preferably the reaction is carried out in the presence of a catalytic amount of an acid, e.g 10 camphorsulfonic acid. Preferred routes to compounds of the invention are shown in the processes below:

### PROCESS 1

1'-Aminospiro[isoquinoline-4(1H),3'-pyrrolidine]-1,2',3,5'(2H)-tetrones of the present invention were prepared by the following reaction scheme: 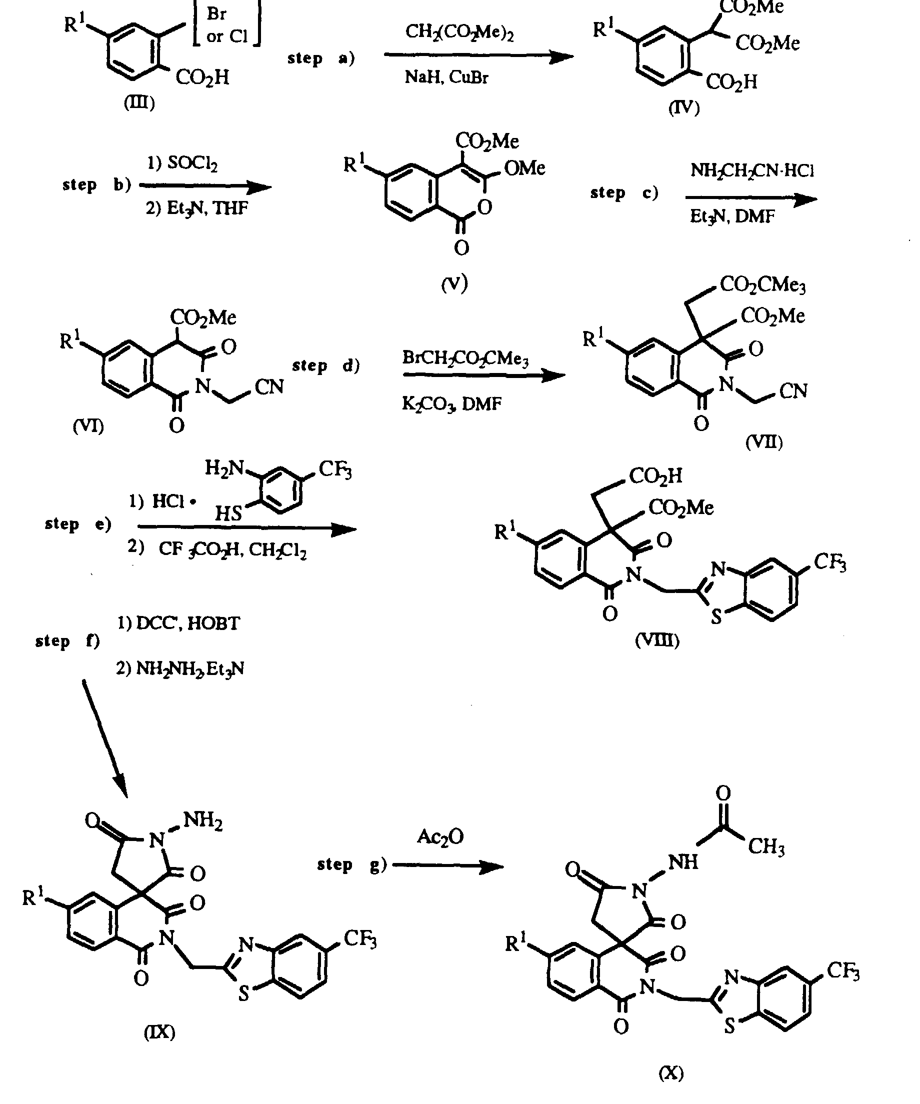 wherein:
R¹ is halogen or hydrogen.

### PROCESS 2

Alkylidene analogs of 1'-amino-2-[(benzothiazolyl)methyl]spiro[isoquinoline-4(1H),3'-pyrrolidine]-1,2',3,5'(2H)-tetrones of the present invention were prepared by the following reaction schem e from the compound of formula IX 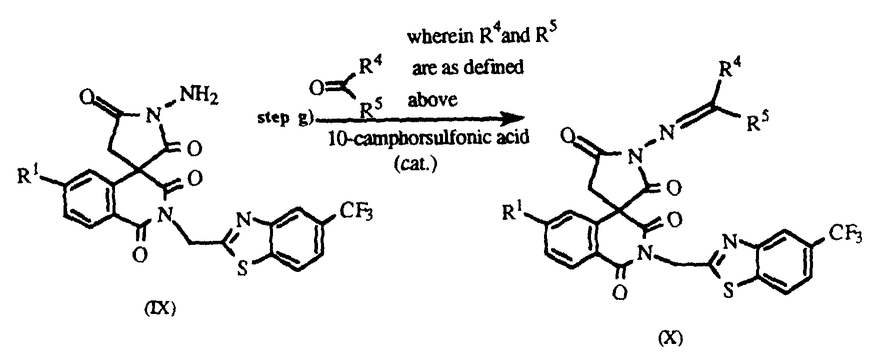 wherein:
R¹ is halogen or hydrogen, R⁴, R5 are are alkyl, or joined to form alicyclic and heterocyclic rings.

### With regard to PROCESS 1:

Step a) Reacting either 2-bromobenzoic acid or 2-chlorobenzoic acid of formula (III) wherein R¹ is as defined above with dimethyl malonate and NaH in the presence of a catalytic amount of CuBr to produce the propanedioic acid dimethyl ester of formula (IV) wherein R¹ is as defined above.
   The 2-bromobenzoic acids or 2-chlorobenzoic acids of formula (III) required for the present invention are commercially available compounds or can be prepared by known methods.
Step b) The propanedioic acid dimethyl ester of formula (IV) can be reacted with thionyl chloride under refluxing conditions to produce the corresponding acid chloride which upon treatment with Et₃N in a conventional solvent which does not adversely influence the reaction, for example, tetrahydrofuran, can produce the compound of formula (V), wherein R¹ is as defined above.
Step c) The compound of formula (V), wherein R¹ is as defined above, is reacted with NH₂CH₂CN·HCl in the presence of Et₃N in a conventional solvent which does not adversely influence the reaction, for example, DMF, produces the compound of the formula (VI), wherein R¹ is as defined above.
Step d) The compound of formula (VI), wherein R¹ is as defined above, is reacted with an inorganic base such as potassium carbonate in a conventional solvent which does not adversely influence the reaction, for example, N,N-dimethylformamide and subsequent addition of the tert-butyl bromoacetate produces the compound of formula (VII), wherein R¹ is as defined above.
Step e) The compound of formula (VII), wherein R¹ is as defined above, can be reacted with 3-amino4-mercaptobenzotrifluoride hydrochloride and an organic acid such as trifluoroacetic acid in a conventional solvent which does not adversely influence the reaction, for example, methylene chloride, to produce the compound of formula (VIII), wherein R¹ is as defined above.
Step f) The compound of formula (VIII), wherein R¹ is as defined above, can be reacted with a coupling agent such as 1-(3-dimethylaminopropyl)-3-ethylcarbodiimide (DCC')/1-hydroxybenzotriazole (HOBT) in a conventional solvent which does not adversely influence the reaction, for example, N,N-dimethylformamide, and subsequent addition of hydrazine and Et₃N, produces the compound of formula (IX), wherein R¹ is as defined above.
Step g) The compound of formula (IX), wherein R¹ is as defined above, can be reacted with acetic anhydride at 70°C, to produce the compound of formula (X), wherein R¹ is as defined above.
With regard to PROCESS 2 the compound of formula (IX), wherein R¹ is as defined above can be reacted with 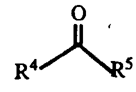 in the presence of a catalytic amount of any acid, for example, 10-camphorsulfonic acid, to produce the compound of formula (X), wherein R¹, R⁴ and R⁵ are as defined above.

The following examples further illustrate this invention:

### Example 1

### 1-'Amino-2-[[5-trifluoromethyl)-2-benzothiazolyl]methyl]spiro[isoquinoline-4(lH),3'-pyrrolidine]-1,2',3,5'(2H)-tetrone

### Step a) (2-Carboxyphenyl)propanedioic Acid Dimethyl Ester

To a rapidly stirred cold suspension (0°C) of 2-bromobenzoic acid (30.0 g, 149.32 mmol), cuprous bromide (2.14 g, 14.93 mmol) and dimethyl malonate (300 mL) was added NaH (80% in mineral oil, 10.75 g, 358.37 mmol) over a 30 minute period, while a stream of dry N₂ was passed over the mixture. After the addition of the NaH had been completed, the mixture was stirred for 10 minutes at room temperature and 30 minutes at 70°C (external oil bath temperature. At this point, the suspension had turned to a solid mass, which was dissolved in H₂O (1000 mL). The aqueous layer was extracted with diethyl ether (3 x 500 mL) and was acidified with HCl (2N). The mixture was extracted with EtOAc and dried over MgSO₄. Evaporation gave an offwhite solid which was recrystallized from Et₂O/hexane (after cooling to -20°C) to give a white solid (34.2 g, 90.9%). ¹H NMR (DMSO-d₆, 400 MHz): δ 3.67 [s, 6H, -CH(CO₂CH₃)₂], 5.72 [s, 1H, -CH(CO₂CH₃)₂], 7.3 (d, J = 7.76 Hz, 1H, Ar-H), 7.45 (dt, J = 7.66 Hz, 1.12 Hz, 1H, Ar-H), 7.6 (dt, J = 7.66 Hz, 1.45 Hz, 1H, Ar-H), 7.94 (dd, J = 7.8 Hz, 1.33 Hz, 1H, Ar-H), 13.2 (s, 1H, -CO₂H); IR (KBr, cm⁻¹); 3300-2700 (CO₂H), 1750 (CO), 1730 (CO), 1680 (CO); MS (m/e): 252 (M⁺), 220 (M⁺-CH₃OH), 188 (M⁺-2 x CH₃OH).

| | | |
|---|---|---|
| Anal. Calcd.: | C, 57.14; | H, 4.80 |
| Found: | C, 57.05; | H; 4.78 |

M.P. 119-120°C.

The following compounds were prepared in substantially the same manner as that of Example 1, Step a):

### (2-Carboxy-5-fluorophenyl)propanedioic Acid Dimethyl Ester

¹H NMR (DMSO-d₆, 400 MHz): δ 3.68 [s, 6H, (-CO₂Me)₂], 5.79 [s, 1H, Ar-CH(CO₂Me)₂], 7.12 (dd, J = 10.06 Hz, 2.61 Hz, 1H, Ar-H), 7.33 (dt, J = 8.48 Hz, 2.64 Hz, 1H, Ar-H), 8.03 (dd, 8.77 Hz, 6.17 Hz, 1H, Ar-H); IR (KBr, cm⁻¹): 3400-2700 (CO₂H), 1730 (CO), 1680 (CO); MS (m/e): 270 (M⁺), 238 (M⁺-CH₃OH), 210 (M⁺-CH₃OH, -CO), 151 (M⁺-CH₃OH, -CO, -CO₂CH₃).

| | | |
|---|---|---|
| Anal. Calcd.: | C, 53.34; | H, 4.10 |
| Found: | C, 53.36; | H, 3.93 |

M.P. 121.5-123.0°C.

### Step b)

### 3-Methoxy-1-oxo-1H-2-benzopyran-4-carboxylic Acid Methyl Ester

A mixture of (2-carboxyphenyl)propanedioic acid dimethyl ester (10.09 g, 39.68 mmol) and SOCl₂ (100g) was refluxed for 2 hours. The volatiles were removed *in vacuo* and the crude product (acid chloride) was dissolved in THF (20 mL). Triethylamine (27.64 mL, 198.4 mmol) was added and the mixture was stirred for 30 minutes. The yellowish suspension was poured into HCl (1N, 1000 mL), extracted with EtOAc and the organic extracts were dried over MgSO₄. Evaporation and crystallization from acetone/ether/hexane (at -20°C) gave a white solid (87.6 g, 94.4%). ¹H NMR (DMSO-d₆, 400 MHz): δ 3.82 (s, 3H, -CO₂Me, 4.03 (s, 3H, -OMe), 7.42 (t, J = 7.26 Hz, 1H, Ar-H), 7.8 (t, J = 8.2 Hz, 1H, Ar-H), 7.9 (d, J = 8.3 Hz, 1H, Ar-H), 8.1 (d, J = 7.26 Hz, 1H, Ar-H); IR (KBr, cm⁻¹): 1740 (C = O), 1685 (C = O); MS (m/e): 234 (16, M⁺), 206 (38.5, M⁺-CO), 203 (12, M⁺-OMe).

| | | |
|---|---|---|
| Anal. Calcd.: | C, 61.59; | H, 4.30 |
| Found: | C, 61.82; | H, 4.29 |

M.P. 129-130°C.

The following compound was prepared in substantially the same manner as that of Example 1, Step b):

### 6-Fluoro-3-methoxy-1-oxo-1 H-2-benzopyran-4-carboxylic Acid Methyl Ester.

¹H NMR (DMSO-d₆, 400 MHz): δ 3.81 (s, 3H, -CO₂CH₃), 4.06 (s, 3H, -OCH3-), 7.27 (dt, J=8.3 Hz, 1H, Ar-H), 7.8 (dd, J = 11.83 Hz, 2.49 Hz, 1H, Ar-H), 8.16 (dd, J = 8.92 Hz, 6.2 Hz, 1H, Ar-H); IR (KBr, cm⁻¹):1750(C = O), 1685 (C = O); MS (rule):252 (24, M⁺), 224 (54, M⁺-CO).

| | | |
|---|---|---|
| Anal. Calcd.: | C, 57.15; | H, 3.60 |
| Found: | C, 57.19; | H, 3.57 |

M.P. 142-143°C.

### Step c)

### 2-(Cyanomethyl)-1,2,3,4-tetrahydro-1,3-dioxo-4-isoquinolinecarboxylic Acid Methyl Ester

To a solution of 3-methoxy-1-oxo-1H-2-benzopyran-4-carboxylic acid methyl ester (8.09 g, 34.19 mmol) in DMF (100 mL) was added aminoacetonitrile hydrochloride (6.32 g, 68.37 mmol) and the suspension was stirred until all the materials had dissolved. Triethylamine (14.3 mL, 102.57 mmol) was added and the mixture was stirred at 100°C for 30 minutes, and then poured into H₂O, acidified with HC (2N) and extracted with EtOAc. The organic extracts were dried over MgSO₄. Evaporation and crystallization from acetone/ether/hexane (at -20°C) gave a yellowish solid (6.5 g, 73.7%). ¹H NMR (DMSO-d₆, 400 MHz): δ (3.7, s, 3.98, s, 3H, -CO₂CH₃, rotameric), (4.92, s, 5.44, s, 2H, -NCH₂CN, rotameric), 7.2-8.4 (m, 4H, Ar-H, rotomeric; IR (KBr, cm⁻¹): 3400 (OH), 1670 (C = O); MS(m/e):258 (20, M⁺), 226 (43, M⁺-MeOH), 199 (13, M⁺-CO₂Me).

| | | | |
|---|---|---|---|
| Anal. Calcd: | C, 60.47; | H, 3.90; | N, 10.85 |
| Found: | C, 60.27; | H, 3.77; | N, 10.69 |

M.P. 169-171°C.

The following compound was prepared in substantially the same manner as that of Example 1, step c)

### 2-(Cyanomethyl)-6-fluoro-1,2,3,4-tetrahydro-1,3-dioxo-4-isoquinolinecarboxylic Acid Methyl Ester

| | | | |
|---|---|---|---|
| Anal. Calcd.: | C, 56.53; | H, 3.28; | N, 10.14 |
| Found: | C, 56.45; | H, 3.22; | N, 10.13 |

M.P.178-179° C.

### Step d)

### 2-(Cyanomethyl)-1,2,3,4-tetrahydro-4-(methoxycarbonyl)-1,3-dioxo-4-isoquinolineacetic Acid 1,1-Dimethylethyl Ester

To a suspension of 2-(cyanomethyl)-1,2,3,4-tetrahydro-1,3-dioxo-4-isoquinoline carboxylic acid methyl ester (6.5 g, 25.19 mmol), K₂CO₃ (6.95 g, 50.38 mmol) and anhydrous DMF (100 mL) was added tert-butyl bromoacetate (6.1 mL, 37.79 mmol). After stirring at 85°C for 3 hours, the mixture was poured into H₂O, acidifed with HCl (2N) and extracted with EtOAc. The organic extracts were dried over MgSO₄. Evaporation and purification by flash chromatography on silica gel (hexane/EtOAc 4/1) gave a white solid (8.5 g, 90.7%).
¹H NMR (DMSO-*d*_{*6*} 200 MHz): δ 1.03 (s, 9H, -CO₂CMe₃), 3.58 (s, 3H, CO₂CH₃), 3.64 (s, 2H, -CH₂CO₂-), 5.05 (s, 2H, -NCH₂CN), 7.64 (m, 2H, Ar-H), 7.78 (dd, J = 7.4 Hz, 2.0 Hz, 1H, Ar-H), 8.24 (dd, J = 8.2 Hz, 1.6 Hz, 1H, Ar-H); IR (KBr, cm⁻¹): 1745 (C = O), 1730 (C = O), 1670 (C = O); MS (CI): 373 (38, M⁺ + H), 317 (100, M⁺+ H,-CMe₃).

| | | | |
|---|---|---|---|
| Anal. Calcd.: | C, 61.28; | H, 5.41; | N, 7.52 |
| Found: | C, 61.61; | H, 5.49; | N, 7.13 |

M.P. 48-50°C.

The following compound was obtained in substantially the same manner as that of Example 1, Step d)

### 2-(Cyanomethyl)-6-fluoro-1,2,3,4-tetrahydro-4-(methoxycarbonyl)-1,3-dioxo-4-isoqinolineacetic Acid 1,1-Dimethylethyl Ester.

| | | | |
|---|---|---|---|
| Anal. Calcd.: | C, 58.46; | H, 4.91; | N, 7.18 |
| Found: | C, 58.65; | H, 4.98; | N, 7.08 |

M.P. 133-135° C.

### Step e)

### 1,2,3,4-Tetrahydro-4-(methoxycarbonyl)-1,3-dioxo-2-[[(5-(trifluoromethyl)-2-benzothiazolyl]methyl]-4-isoquinolineacetic Acid

To a mixture of 3-amino-4-mercaptobenzotrifluoride hydrochloride (6.1 g, 26.2 mmol) and EtOH (150 mL) was added Et₃N (3.65 mL). After stirring for 10 minutes, 2-(cyanomethyl)-1,2,3,4-tetrahydro-4-(methoxycarbonyl)-1,3-dioxo-4-isoquinolineacetic acid 1,1-dimethylethyl ester (6.5 g, 17.47 mmol) was added and the mixture was refluxed for 15 hours, poured into H₂O, acidified with HCl (2N) and extracted with EtOAc. The organic extracts were dried over MgSO₄. Evaporation gave an oil (9.6 g) which was dissolved in CH₂Cl₂ (80 mL). Trifluoroacetic acid (20 mL) was added and the mixture was stirred at room temperature for 8 hours. The volatiles were removed *in vacuo* and the residue was purified by flash chromatography on acid washed (5%, H₃PO₄ in MeOH) silica gel to give a white solid (6.3 g, 73.3%).
¹H NMR (DMSO-*d*_{*6*}, 400 MHz) δ 3.57 (s, 3H, -CO₂CH₃), 3.68 (dd, J = 17.85 Hz, 2H, -CH₂CO₂H), 5.61 (s, 2H, -NCH₂-), 7.62 (m, 2H, Ar-H), 7.81 (m, 2H, Ar-H), 8.2 (dd, J = 7.9 Hz, 1.04 Hz, 1H, Ar-H), 8.33 (dd, J = 8.5 Hz, 0.92 Hz, 1H, Ar-H), 8.34 (d, J = 083 Hz, 1H, Ar-H); IR (KBr, cm⁻¹):3200-2500 (CO₂H), 1750 (C = O), 1710 (C = O), 1670 (C = O); MS(m/e):492 (6, M⁺), 448 (6, M⁺-CO2), 416 (62, M⁺-CO₂-MeOH).

| | | | |
|---|---|---|---|
| Anal. Calcd.: | C, 53.66; | H, 3.07; | N, 5.69 |
| Found: | C, 53.40; | H, 3.01; | N, 5.54 |

M.P.199-201° C.

The following compound was prepared in substantially the same manner as that of Example 1, step e).

### 6-Fluoro-1,2,3,4-tetrahydro-4-(methoxycarbonyl)-1,3-dioxo-2-[[(5-trifluoromethyl)-2-benzothiazolyl]methyl]-4-isoquinolineacetic Acid

| | | | |
|---|---|---|---|
| Anal. Calcd.: | C, 51.77; | H, 2.76; | N, 5.49 |
| Found: | C, 51.62; | H, 2.97; | N, 5.18 |

### Step f)

### 1'-Amino-2-[[5-(trifluoromethyl)-2-benzothiazolyl]methyl]spiro[isoquinoline-4(1H),3'-pyrrolidine]-1,2',3,5'(2H)-tetrone

To a solution of 1,2,3,4,-tetrahydro-4-(methoxycarbonyl)-1,3-dioxo-2-[[5-(trifluoromethyl)-2-benzothiazolyl]methyl acid (12.0 g, 24.39 mmol) in DMF (200 mL) were added 1-(3-dimethylaminopropyl)-3-ethylcarbodiimide hydrocholoride (DCC', 6.0 g, 31.7 mmol) and 1-hydroxybenzotriazole hydrate (HOBT, 4.94 g, 36.58 mmol). After stirring for 2 hours, anhydrous hydrazine (0.99 mL, 31.7 mmol) was added dropwise, followed by Et₃N (6.8 mL, 48.78 mmol) addition. The mixture was stirred for 30 minutes, poured into H₂O and extracted with EtOAc. The organic extracts were dried over MgSO₄. Evaporation and purification by flash chromatography (hexane/EtOAc 1:1) gave a white solid (9.6 g, 83.0%).
¹H NMR (DMSO-d₆, 400MHz): δ 3.42 (d, J = 18.2 Hz, 1H, -HCHCO-), 3.57 (d, J = 18.2 Hz, 1H, -HCHCO-), 5.25 (s, 2H, -NH₂-), 5.57 (s, 2H, -NCH₂), 7.6 (d, J = 7.9 Hz, 1H, Ar-H), 7.67 (t, J = 7.7 Hz, 1H, Ar-H), 7.77 (dd, J = 8.5 Hz, 1.7 Hz, 1H, Ar-H), 7.82 (dt, J = 7.5 Hz, 1.45 Hz, 1H, Ar-H), 8.22 (dd, J = 7.7 Hz, 1.04 Hz, 1H, Ar-H), 8.3 (s, 1H, Ar-H), 8.35 (d, J = 8.9 Hz, 1H, Ar-H); IR (KBr, cm⁻¹): 3420 (NH₂), 1715 (C=O), 1670 (C=O); MS (m/e): 475 (88, M+H)⁺.

| | | | |
|---|---|---|---|
| Anal. Calcd.: | C, 53.17; | H, 2.76; | N, 11.81 |
| Found: | C, 53.05; | H, 2.74; | N, 12.13 |

M.P. 116-118°C.

The following compound was prepared in substantially the same manner as that of Example 1, step f).

### 1'-Amino-6-fluoro-2-[[5-(trifluoromethyl)-2-benzothiazolyl]methylspiro[isoquinoline-4(1H),3'-pyrrolidine]-1,2',3,5'(2H)-tetrone

¹H NMR (DMSO-d₆, 400 MHz): δ 3.44 (d, J = 18.2 Hz, 1H, -HCHCO-), 3.48 (d, J = 18.2 Hz, 1H, -HCHCO-), 5.2 (s, 2H, -NH₂), 5.55 (s, 2H, -NCH₂), 7.52 (dt, J = 8.5 Hz, 2.3 Hz, 1H, Ar-H), 7.6 (dd, 7.5 Hz, 2.5 Hz, 1H, Ar-H), 7.77 (dd, J = 8.3 Hz, 1.45 Hz, 1H, Ar-H), 8.27-8.35 (m, 3H, Ar-H); IR (KBr, cm⁻¹): 3420 (NH₂), 1720 (C=O), 1670 (C=O); MS (m/e): (100, M+H)⁺.

| | | | |
|---|---|---|---|
| Anal. Calcd.: | C, 51.22; | H, 2.46; | N, 11.38 |
| Found: | C, 51.20; | H, 2.42; | N, 11.28 |

M.P. 118-120°C.

### Example 2

### 1'-[(1-Methylethylidene)amino]-2-[[5-(trifluoromethyl)-2-benzothiazolyl]methyl]spiro[isoquinoline]-1,2',3,5'(2H)-tetrone

A mixture of 1'-amino-2-[[5-(trifluoromethyl)-2-benzothiazolyl]methyl]spiro[isoquinoline-4(1H),3'-pyrrolidine]-1,2',3,5'(2H)-tetrone (2.0 g, 4.22 mmol), acetone (20 mL) and 10-camphorsulfonic acid (30 mg) was refluxed for 30 minutes. The volatiles were removed *in vacuo* and the residue was purified by flash chromatography on silica gel (hexane/EtOAc 1:1) to give a white solid (1.69 g, 77%). ¹H NMR (DMSO-d₆, 400 MHz): δ 1.78 (s, 3H, -CH₃), 2.15 (s, 3H, -CH₃), 3.6 (s, 2H, -CH₂CO-), 5.57 (s, 2H, -NCH₂), 7.68 (dt, J = 7.9 Hz, 1.7 Hz, 1H, Ar-H), 7.75 (dd, J = 8.5 Hz, 1.45 Hz, 1H, Ar-H), 7.82-7.86 (m, 2H, Ar-H), 8.22 (d, J = 7.9 Hz, 1H, Ar-H), 8.3 (s, 1H, Ar-H), 8.33 (d, J = 8.5 Hz, 1H, Ar-H); IR (KBr, cm⁻¹): 1715 (C=O), 1670 (C=O); MS (m/e): 515 (100, M+H)⁺.

| | | | |
|---|---|---|---|
| Anal. Calcd.: | C, 56.03; | H, 3.33; | N, 10.89 |
| Found: | C, 56.22; | H, 3.47; | N, 10.75 |

M.P. 121-123°C.

The following compounds were prepared in substantially the same manner as that of Example 2 a)

### 1'-(Cyclopentylideneamino)-2-[[5-(trifluoromethyl)-2-benzothiazolyl]methyl]spiro[isoquinoline-4(1H),3'pyrrolidine]-1,2',3,5'(2H)-tetrone

¹H NMR (DMSO-d₆, 400 MHz): δ 1.45-1.8 (m, 4H, -CH₂CH₂-), 2.0-2.2 (m, 2H, -CH₂-), 2.57 (m, 2H, -CH₂), 3.58 (s, 2H, -CH₂CO-), 5.51 (d, J = 16.2 Hz, 1H, -NHCH-), 5.59 (d, J = 16.2 Hz, 1H, -NHCH-), 7.68 (dt, J = 7.9 Hz, 1.45 Hz, 1H, Ar-H), 7.75 (dd, J = 8.5 Hz, 1.45 Hz, 1H, Ar-H), 7.8-7.87 (m, 2H, Ar-H), 8.22 (d, J = 7.9 Hz, 1H, Ar-H), 8.28 (s, 1H, Ar-H), 8.33 (d, J = 8.3 Hz, 1H, Ar-H); IR (KBr, cm⁻¹): 1710 (C=O), 1675 (C=O); MS (m/e): 541 (18, M+H)⁺.

| | | | |
|---|---|---|---|
| Anal. Calcd.: | C, 57.77; | H, 3.52; | N, 10.37 |
| Found: | C, 58.00; | H, 3.52; | N, 10.45 |

M.P. 178-180°C.

### 1'-[(Tetrahydro-4H-pyran-4-ylidene)amino]-2-[[5-(trifluoromethyl)-2-benzothiazolyl]methyl]spiro[isoquinoline-4(1H),3'-pyrrolidine]-1,2',3,5'(2H)-tetrone

¹H NMR (DMSO-d₆, 400 MHz): δ 2.23 (t, J = 5.8 Hz, 2H, -CH₂CH₂O), 2.57 (t, J = 5.8 Hz, 2H, -CH₂CH₂O), 3.5 (m, 1H, -HCHO-), 3.6 (m, 3H, -HCHO-, -CH₂CO-), 3.72-3.85 (m, 2H, -CH₂O-), 5.51 (d, J = 16.0 Hz, 1H, -HCHN-), 5.58 (J = 16.0 Hz, 1H, -HCHN-), 7.6 (m, 1H, Ar-H), 7.77 (dd, J = 8.5 Hz, 1.87 Hz, 1H, Ar-H), 7.85 (m, 2H, Ar-H), 8.22 (d, J = 7.9 Hz, 1H, Ar-H), 8.3 (s, 1H, Ar-H), 8.35 (d, J = 8.5 Hz, 1H, Ar-H); IR (KBr, cm⁻¹): 1720 (C=O), 1670 (C=O); MS (m/e): 557 (80, M+H)⁺.

| | | | |
|---|---|---|---|
| Anal. Calcd.: | C, 56.11; | H, 3.44; | N, 10.07 |
| Found: | C, 55.86; | H, 3.34; | N, 10.05 |

M.P. 191-193°C.

### EXAMPLE 3

### N-[2-[[5-(trifluoromethyl)-2-benzothiazolyl]methyl]-2,3-dihydro-1,2',3,5'-tetraoxospiro[isoquinoline-4(1H),3'-pyrrolidine]-1'-yl] acetamide

A mixture of 1'-amino-2-[[5-(trifluoromethyl)-2-benzothiazolyl]methyl]spiro[isoquinoline-4(1H),3'-pyrrolidine]-1,2',3,5'(2H)-tetrone (2.0 g, 4.32 mmol) and acetic anhydride (20 mL) was stirred at 80°C for 1 hour. The volatiles were removed *in vacuo* and the residue was purified by flash chromatography on silica gel (hexane/EtOAc 1:1) to give a white solid (1.0, 83.8%).
¹H NMR (DMSO-d₆, 400 MHz): δ 2.0 (s, 3H, -COCH₃), 3.53 (d, J = 18.2 Hz, 1H, -HCHCO-), 3.73 (d, J = 18.2 Hz, 1H, -HCHCO-), 5.55 (s, 2H, -NCH₂-), 7.6 (d, J = 7.9 Hz, 1H, Ar-H), 7.7 (t, J = 7.9 Hz, 1H, Ar-H), 7.77 (dd, 8.3 Hz, 1.45 Hz, 1H, Ar-H), 7.86 (t, J = 7.3 Hz, 1H, Ar-H), 8.23 (d, J = 7.7 Hz, H, Ar-H), 8.3 (s, 1H, Ar-H), 8.35 (d, J = 8.5 Hz, 1H, Ar-H), 11.0 (s, 1H, -NNHCOCH₃); IR (BKr, cm⁻¹): 3420 (NH), 1740 (C=O), 1675 (C=O); MS (m/e): (100, M+H)⁺.

| | | | |
|---|---|---|---|
| Anal. Calcd.: | C, 53.49; | H, 2.93; | N, 10.85 |
| Found: | C, 53.23; | H, 2.80; | N, 10.75 |

M.P. 142-144°C.

## Claims

1. A compound of formula: 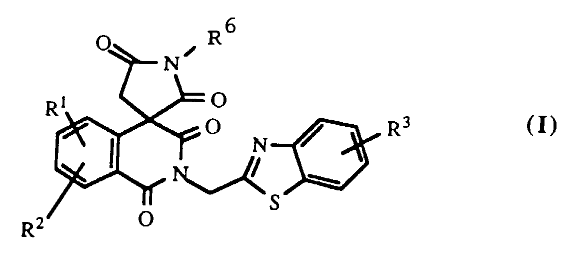 wherein:
R¹,R² and R³ are independently hydrogen, alkyl containing 1 to 6 carbon atoms, halogen, lower alkoxy containing 1 to 6 carbon atoms, trifluoromethyl or nitro;
R⁶ is 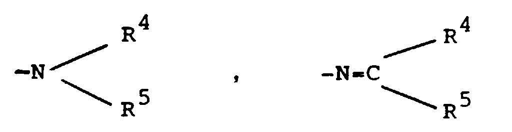 or 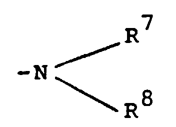 wherein
R⁴ and R⁵ are independently hydrogen, alkyl containing 1 to 6 carbon atoms, aryl, aryl (lower alkyl) wherein aryl contains 6 to 10 carbon atoms and lower alkyl contains 1 to 6 carbon atoms, alkanoyl of 2 to 5 carbon atoms or carboalkoxy, or R⁴ and R⁵ are joined to form alicyclic or heterocyclic rings selected from the group consisting of 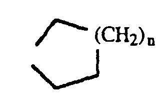 wherein n=1-10; 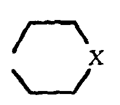 wherein X = O, S, SO, SO₂; 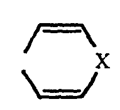 wherein X = O, S; and 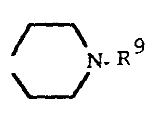 wherein R⁹ = H, lower alkyl containing 1 to 6 carbon atoms, aryl or aryl flower alkyl)wherein aryl contain 6 to 10 carbon atoms and lower alkyl contains 1 to 6 carbon atoms,
and
R⁷ and R⁸ are independently alkylsulfoxy, arylsulfoxy, alkylsulfonyl, arylsulfonyl wherein aryl contains 6 to 10 carbon atoms and alkyl contains 1 to 6 carbon atoms or R⁸ may also represent one of the values for R⁵ above, and the pharmaceutically acceptable salts thereof.

2. A compound according to Claim 1 of structural formula 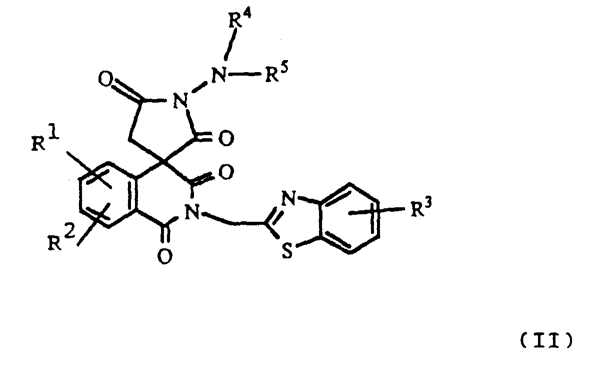 wherein:
R¹ and R² are hydrogen or halogen; R³ is trifluoromethyl; R⁴ and R⁵ are hydrogen or acetyl.

3. A compound according to Claim 1 of structural formula (IIa) 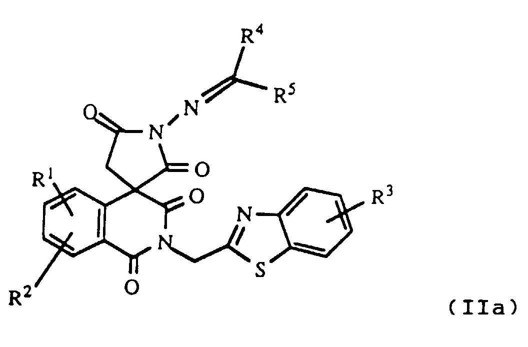 wherein:
R¹ and R² are hydrogen or halogen; R³ is trifluoromethyl; R⁴ and R⁵ are lower alkyl containing 1 to 3 carbon atoms or R⁴ and R⁵ are joined to form alicyclic or heterocyclic rings selected from the group consisting of

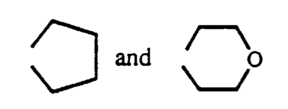

4. The compound according to Claim 3 1'-[(1-methylethylidene)amino]-2-[[5-(trifluoromethyl)-2-benzothiazolyl]methyl]spiro[isoquinoline-4(1H),3'-pyrrolidine]-1,2',3,5'(2H)-tetrone.

5. The compound according to Claim 3 1'-(cyclopentylideneamino)-2-[[5-(trifluoromethyl)-2-benzothiazolyl]methyl]spiro[isoquinoline-4(1H),3'-pyrrolidine]-1,2',3'5'(2H)-tetrone.

6. The compound according to Claim 3 1'-[(tetrahydro-4H-pyran-4-ylidene)amino-2-[[5-(trifluoromethyl)-2-benzothiazolyl]methyl]spiro-[isoquinoline-4(1H),-3'-pyrrolidine]-1,2',3,5'(2H)-tetrone.

7. The compound according to Claim 2 1'-amino-2-[[5-(trifluoromethyl)-2-benzothiazolyl]methyl]spiro[isoquinoline-4(1H),3'-pyrrolidine]-1,2',3,5'(2H)-tetrone.

8. The compound according to Claim 2 1'-amino-6-fluoro-2-[[5-(trifluoromethyl)-2-benzothiazolyl]methyl]spiro[isoquinoline-4(lH),3'-pyrrolidine]-1,2',3,5'(2H)-tetrone.

9. The compound according to Claim 2 N-[2-[[5-(trifluoromethyl)-2-benzothiazolyl]methyl]-2,3-dihydro-1,2',3,5'-tetraoxospiro[isoquinoline-4(1H),3'-pyrrolidine]-1'-yl]acetamide.

10. A process for preparing a compound as claimed in Claim 1 which comprises one of the following:
a) acylating a compound of formula (A) or (B)
R⁴R⁵NNH₂ (A)
R⁸R⁷NNH₂ (B)
wherein R⁴, R⁵, R⁸ and R⁷ are as defined in Claim 1 with a compound of formula 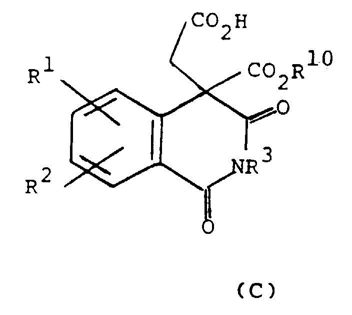 or an activated form thereof
wherein CO₂R¹⁰ and is an ester function and R¹, R² and R³ are as defined in Claim 1 to give a corresponding compound of formula I wherein R⁶ is -NR⁴R⁵ or -NR⁸R⁷ ,
or
b) acylating a compound of formula I as defined in Claim 1 wherein R⁶ is -NHR⁸
with an acylating agent containing the group
R¹¹CO- ,
R¹²OCO- ,
or
R¹³S(O)ₘ
wherein m is 1 or 2, R¹ is C₁-C₆ alkyl,
R¹² iS C₁-C₆ alkyl and R¹³ is C₁-C₆ alkyl, or C₆-C₁₀ aryl to give a corresponding compound of formula I wherein R⁴ is alkanoyl orcarboalkoxy, and R⁷ is alkylsulfoxy, alkysulfonyl, arylsulfoxy or arylsulfonyl or
c) reacting a compound of formula I wherein R⁶ is NH₂
with a carbonyl compound of formula (D): 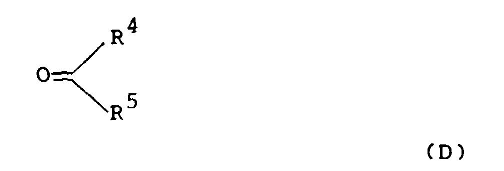 wherein R⁴ and R⁵ are as defined above, or a reactive derivative thereof, e.g an acetal, to give a compound of formula I wherein R⁶ is
-N=CR⁴R⁵
and if desired after any of the aforementioned processes isolating the product as a salt.

11. A pharmaceutical composition comprising a compound of formula I or a pharmaceutically acceptable salt thereof as claimed in Claim 1 and a pharmaceutically acceptable carrier.

## Patentansprüche

1. Verbindung der Formel: 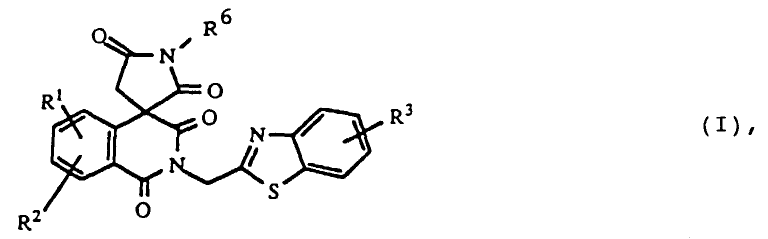 worin R¹, R² und R³ unabhängig Wasserstoff, Alkyl mit 1 bis 6 Kohlenstoffatomen, Halogen, nied.Alkoxy mit 1 bis 6 Kohlenstoffatomen, Trifluormethyl oder Nitro bedeuten;
R⁶ 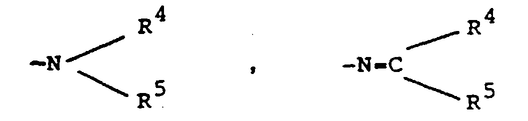 oder 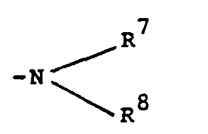 darstellt, wobei R⁴ und R⁵ unabhänging Wasserstoff, Alkyl mit 1 bis 6 Kohlenstoffatomen, Aryl, Aryl-(nied.alkyl), worin Aryl 6 bis 10 Kohlenstoffatome enthält, und nied.Alkyl 1 bis 6 Kohlenstoffatome enthält, Alkanoyl mit 2 bis 5 Kohlenstoffatomen oder Carboalkoxy sind, oder R⁴ und R⁵ verbunden sind, um alicyclische oder heterocyclische Ringe zu bilden, ausgewählt aus der Gruppe bestehend aus 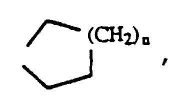 worin n = 1 bis 10; 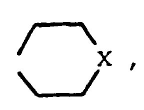 worin X = O, S, SO, SO₂; 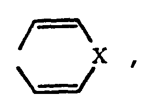 worin X = O, S; und 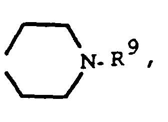 worin R⁹ = H, nied.Alkyl mit 1 bis 6 Kohlenstoffatomen, Aryl oder Aryl(nied.alkyl), wobei Aryl 6 bis 10 Kohlenstoffatome enthält, und nied.Alkyl 1 bis 6 Kohlenstoffatome enthält;
und R⁷ und R⁸ unabhängig Alkylsulfoxy, Arylsulfoxy, Alkylsulfonyl, Arylsulfonyl bedeuten, wobei Aryl 6 bis 10 Kohlenstoffatome enthält, und Alkyl 1 bis 6 Kohlenstoffatome enthält, oder R⁸ auch einen der Werte für R⁵ oben darstellen kann, und die pharmazeutisch annehmbaren Salze hievon.

2. Verbindung nach Anspruch 1 der Strukturformel 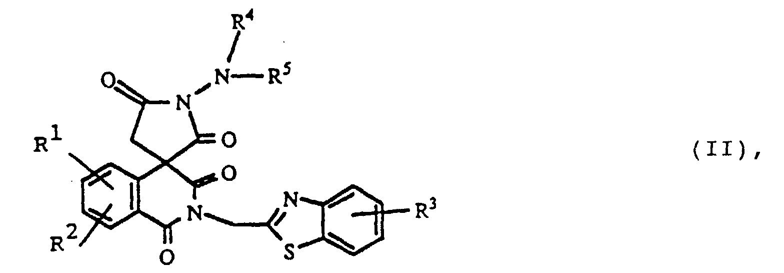 worin R¹ und R² Wasserstoff oder Halogen bedeuten; R³ Trifluormethyl darstellt; R⁴ und R⁵ Wasserstoff oder Acetyl sind.

3. Verbindung nach Anspruch 1 der Strukturformel (IIa) 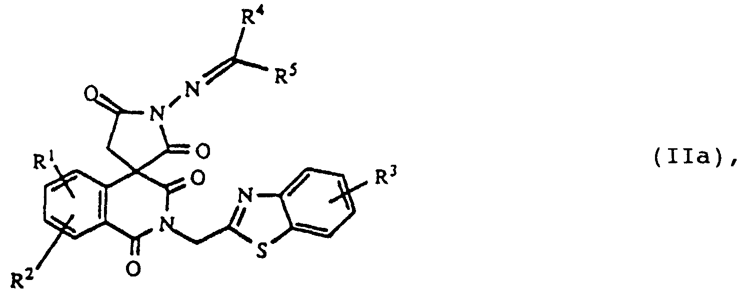 worin R¹ und R² Wasserstoff oder Halogen bedeuten; R³ Trifluormethyl darstellt; R⁴ und R⁵ nied.Alkyl mit 1 bis 3 Kohlenstoffatomen sind, oder R⁴ und R⁵ verbunden sind, um alicyclische oder heterocyclische Ringe zu bilden, ausgewählt aus der Gruppe bestehend aus

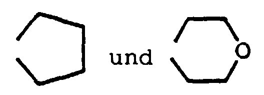

4. Verbindung nach Anspruch 3
1'-[(1-Methylethyliden)-amino]-2-[[5-(trifluormethyl)-2-benzothiazolyl]-methyl]-spiro-[isochinolin-4(1H),3'-pyrrolidin]-1,2',3,5'(2H)-tetron.

5. Verbindung nach Anspruch 3
1'-(Cyclopentylidenamino)-2-[[5-(trifluormethyl)-2-benzothiazolyl]-methyl]-spiro-[isochinolin-4(1H),3'-pyrrolidin]-1,2',3,5'(2H)-tetron.

6. Verbindung nach Anspruch 3
1'-[(Tetrahydro-4H-pyran-4-yliden)-amino]-2-[[5-(trifluormethyl)-2-benzothiazolyl]-methyl]-spiro-[isochinolin-4(1H),3'-pyrrolidin]-1,2',3,5'(2H)-tetron.

7. Verbindung nach Anspruch 2
1'-Amino-2-[[5-(trifluormethyl)-2-benzothiazolyl]-methyl]-spiro[isochinolin-4(1H),3'-pyrrolidin]-1,2',3,5'(2H)-tetron.

8. Verbindung nach Anspruch 2
1'-Amino-6-fluor-2-[[5-(trifluormethyl)-2-benzothiazolyl]methyl]-spiro-[isochinolin-4(1H),3'-pyrro1idin]-1,2',3,5'(2H)-tetron.

9. Verbindung nach Anspruch 2
N-[2-[[5-(Trifluormethyl)-2-benzothiazolyl]-methyl]-2,3-dihydro-1,2',3,5'-tetraoxospiro-[isochinolin-4(1H),3'-pyrrolidin]-1'-yl]-acetamid.

10. Verfahren zur Herstellung einer Verbindung nach Anspruch 1, welches eines der folgenden umfaßt:
a) Acylieren einer Verbindung der Formel (A) oder (B)
R⁴R⁵ NNH₂ (A)
R⁸R⁷NNH₂ (B),
worin R⁴, R⁵, R⁸ und R⁷ wie in Anspruch 1 definiert sind, mit einer Verbindung der Formel 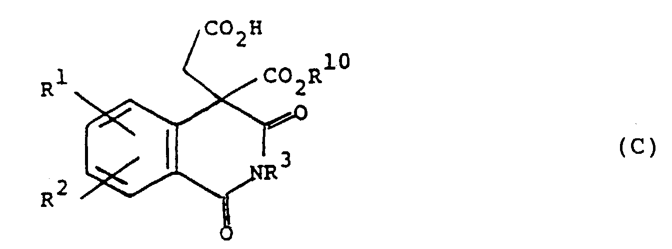 oder einer aktivierten Form hievon, worin CO₂R¹⁰ eine Ester-Funktion bedeutet, und R¹, R² und R³ wie in Anspruch 1 definiert sind, wobei eine entsprechende Verbindung der Formel (I) erhalten wird, worin R⁶ -NR⁴R⁵ oder -NR⁸R⁷ darstellt; oder
b) Acylieren einer Verbindung der Formel (I), wie in Anspruch 1 definiert, worin R⁶ -NHR⁸ bedeutet, mit einem Acylierungsmittel, das die Gruppe
R¹¹CO-,
R¹²OCO-
oder
R¹³S(O)ₘ
enthält, wobei m 1 oder 2 ist, R¹¹ C₁-C₆-Alkyl bedeutet, R¹² C₁-C₆-Alkyl darstellt, und R¹³ C₁-C₆-Alkyl oder C₆-C₁₀-Aryl ist, wobei eine entsprechende Verbindung der Formel (I) erhalten wird, worin R⁴ Alkanoyl oder Carboalkoxy darstellt, und R⁷ Alkylsulfoxy, Alkylsulfonyl, Arylsulfoxy oder Arylsulfonyl ist; oder
c) Umsetzen einer Verbindung der Formel (I), worin R⁶ NH₂ bedeutet, mit einer Carbonyl-Verbindung der Formel (D): 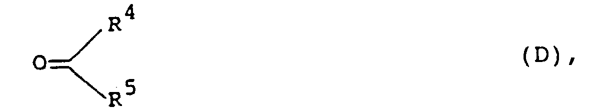 worin R⁴ und R⁵ wie oben definiert sind, oder einem reaktiven Derivat hievon, z.B. einem Acetal, wobei eine Verbindung der Formel (I) erhalten wird, worin R⁶
-N=CR⁴R⁵
bedeutet;
und, wenn gewünscht, nach einem beliebigen der oben angegebenen Verfahren, Isolieren des Produkts als Salz.

11. Pharmazeutische Zusammensetzung, welche eine Verbindung der Formel (I) oder ein pharmazeutisch annehmbares Salz hievon nach Anspruch 1 und einen pharmazeutisch annehmbaren Träger umfaßt.

## Revendications

1. Composé de formule : 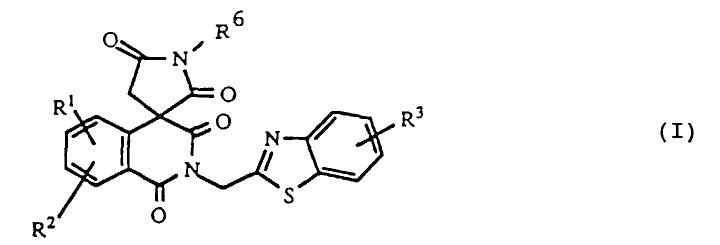 dans laquelle :
R¹, R² et R³ sont indépendamment, hydrogène, alcoyle contenant 1 à 6 atomes de carbone, halogène, alcoxy inférieur contenant 1 à 6 atomes de carbone, trifluorométhyle ou nitro;
R⁶ est 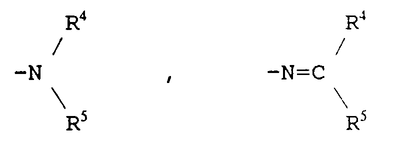 ou 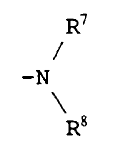 où
R⁴ et R⁵ sont indépendamment, hydrogène, alcoyle contenant 1 à 6 atomes de carbone, aryle, aryl(alcoyle inférieur) où aryle contient 6 à 10 atomes de carbone et alcoyle inférieur contient 1 à 6 atomes de carbone, alcanoyle de 2 à 5 atomes de carbone ou carboalcoxy, ou R⁴ et R⁵ sont joints pour former des cycles alicycliques ou hétérocycliques choisis parmi le groupe consistant en 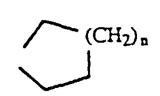 où n=1-10; 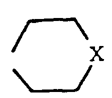 où X=0, S, SO, SO₂; 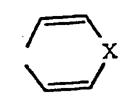 où X=O, S; et 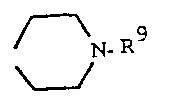 où R⁹=H, alcoyle inférieur contenant 1 à 6 atomes de carbone, aryle ou aryl(alcoyle inférieur) où aryle contient 6 à 10 atomes de carbone et alcoyle inférieur contient 1 à 6 atomes de carbone, et
R⁷ et R⁸ sont indépendamment, alcoylsulfoxy, arylsulfoxy, alcoylsulfonyle, arylsulfonyle, où aryle contient 6 à 10 atomes de carbone et alcoyle contient 1 à 6 atomes de carbone ou R⁸ peut également représenter l'une des valeurs de R⁵ définies ci-dessus, et les sels pharmaceutiquement acceptables de ceux-ci.

2. Composé suivant la revendication 1 de formule de structure : 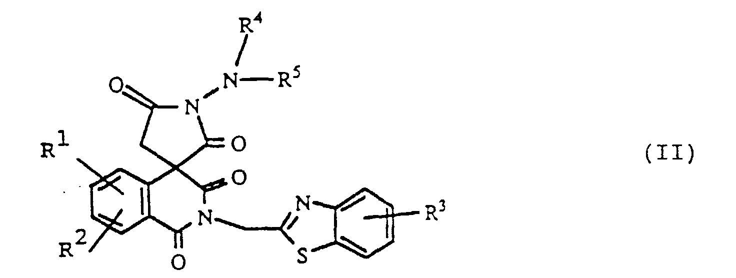 dans laquelle :
R¹ et R² sont hydrogène ou halogène; R³ est trifluorométhyle; R⁴ et R⁵ hydrogène ou acétyle.

3. Composé suivant la revendication 1 de formule de structure (IIa) : 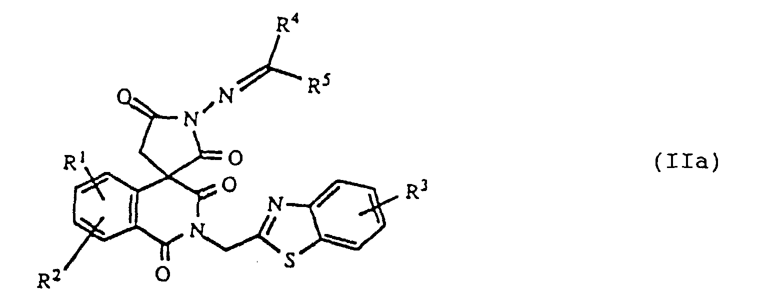 dans laquelle :
R¹ et R² sont hydrogène ou halogène; R³ est trifluorométhyle; R⁴ et R⁵ sont alcoyle inférieur contenant 1 à 3 atomes de carbone, ou R⁴ et R⁵ sont joints pour former des cycles alicycliques ou hétérocycliques choisis parmi le groupe consistant en

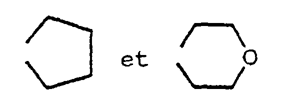

4. Composé suivant la revendication 3, la 1'-[(1-méthyléthylidène)amino]-2-[[5-(trifluorométhyl)-2-benzothiazolyl]méthyl]spiro[isoquinoléine-4(1H), 3'-pyrrolidinej-1,2',3,5'(2H)-tétrone.

5. Composé suivant la revendication 3, la 1'-(cyclopentylidèneamino)-2-[[5-(trifluorométhyl)-2-benzothiazolyljnéthyl]spiro[isoquinoléine-4(1H),3'-pyrrolidine]-1,2',3 ,5'(2H)-tétrone.

6. Composé suivant la revendication 3, la 1'-[tétrahydro-4H-pyran-4-ylidène)amino-2-[[5-(trifluorométhyl)-2-benzothiazolyl]méthyl]spiro[isoquinoléine-4(1H),3'-pyrrolidine]-1,2',3,5'(2H)-tétrone.

7. Composé suivant la revendication 2, la 1'-amino-2-[[5-(trifluorométhyl)-2-benzothiazolyl]méthyl]spiro[isoquinoléine-4(1H),3'-pyrrolidine]1,2',3,5'(2H)-tétrone.

8. Composé suivant la revendication 2, la 1'-amino-6-fluoro-2-[[5-(trifluorométhyl)-2-benzothiazolyl]méthyl]spiro[isoquinoléine-4(1H),3'-pyrrolidine]-1,2',3,5'(2H)-tétrone.

9. Composé suivant la revendication 2, le N-[2-[[5-(trifluorométhyl)-2-benzothiazolyl]méthyl]-2,3-dihydro-1,2',3,5'-tétraoxospiro[isoquinoiéine-4(1H),3'-pyrrolidine]-1'-yl]acétamide.

10. Procédé de préparation d'un composé suivant la revendication 1, qui comprend l'un des suivants :
a) acylation d'un composé de formule (A) ou (B)
R⁴R⁵NNH₂ (A)
R⁸R⁷NNH₂ (B)
où
R⁴, R⁵, R⁸ et R⁷ sont tels que définis à la revendication 1, avec un composé de formule : 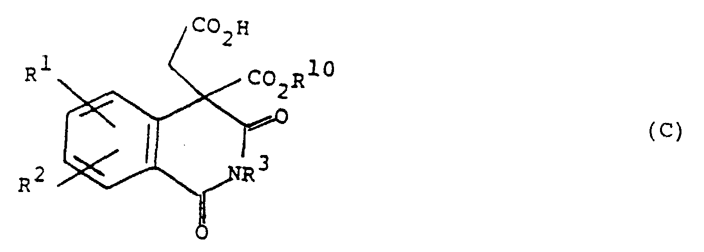 ou une forme activée de celui-ci,
dans lequel CO₂R¹⁰ est une fonction ester et R¹, R² et R³ sont tels que définis à la revendication 1, pour donner un composé correspondant de formule (I) dans lequel R⁶ est -NR⁴R⁵ ou -NR⁸R⁷,
ou
b) acylation d'un composé de formule (I), comme défini à la revendication 1, dans lequel R⁶ est -NHR⁸, avec un agent acylant contenant le groupe
R¹¹CO-, R¹²OCO- ou R¹³S(O)ₘ-
où
m est 1 ou 2, R' est alcoyle en C₁-C₆,
R¹² est alcoyle en C₁-C₆ et R¹³ est alcoyle en C₁-C₆ ou aryle en C₆-C₁₀, pour donner un composé correspondant de formule (I) dans lequel R⁴ est alcanoyle ou carbonalcoxy, et R⁷ est alcoylsulfoxy, alcoylsulfonyle, arylsulfoxy ou arylsulfonyle,
ou
c) réaction d'un composé de formule (I) dans lequel R⁶ est -NH₂, avec un composé carbonylé de formule (D) : 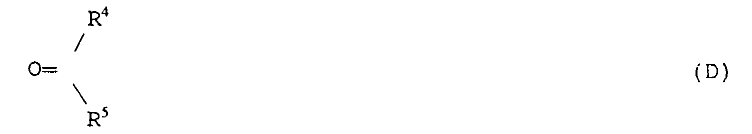 où
R⁴ et R⁵ sont tels que définis ci-dessus, ou un dérivé réactif de celui-ci, par exemple un acétal, pour donner un composé de formule (I) dans lequel R⁶ est
-N=CR⁴R⁵
et si souhaité, après l'un quelconque des procédés prémentionnés, isolement du produit sous forme d'un sel.

11. Composition pharmaceutique comprenant un composé de formule (I) ou un sel pharmaceutiquement acceptable de celui-ci, suivant la revendication 1, et un excipient pharmaceutiquement acceptable.
